# EUROPEAN PATENT APPLICATION

(11) **EP 1 980 265 A1**
(43) Date of publication of application: **15.10.2008**
(21) Application number: 07706768.4
(22) Date of filing: 15.01.2007
(51) Int. Cl.: A61K 38/48, A61P 35/00, A61P 43/00, A61P 9/04, A61P 9/12

(54) **THERAPEUTIC AGENT**

(30) Priority: 17.01.2006 JP 2006009276; 24.03.2006 JP 2006083522
(71) Applicant: P-Lap Corporation, Nagoya-shi, Aichi 465-0032 (JP)
(72) Inventor: MIZUTANI, Shigehiko, Aichi 4650032 (JP)
(74) Representative: Prüfer & Partner GbR European Patent Attorneys
(86) International application number: PCT/JP2007/050434
(87) International publication number: WO 2007/083603

(57) **Abstract**

[Object] To provide a therapeutic agent which is effective and practical regardless of the content of the diseases without affecting the mother's body or the fetus of a patient suffering from pregnancy hypertension.

[Solving Means] An APA of a secretion type prepared by substitution of the amino acid sequence from N terminal to the 71st one with a signal peptide of human trypsin II or of human A-LAP was incubated utilizing insect cells whereupon a human APA protein was produced. Then, the resulting human APA protein was orally administered to a naturally hypertensive rat and changes in the mean blood pressure thereafter were changed whereupon it was found that the blood pressure effectively lowered within short time and, further, the low blood pressure was maintained for long time by a sole administration or a continuous administration of the APA protein.

## Description

### Technical Field

The present invention relates to a therapeutic agent for various kinds of diseases and, more particularly, it relates to a therapeutic agent having efficacy to hypertension and especially to pregnancy hypertension (gestational hypertension).

### Background Art

The birth numbers in Japan are in a decreasing tendency in recent years while birth numbers of premature babies (low birth weight infants) where body weight is not more than 2, 500 g are increasing. As a cause therefor, increases in the cases of gestational hypertension and threatened premature delivery due to aging of pregnant women and to changes in social structure are exemplified. As to a therapeutic agent for the pregnancy hypertension as such, hydrazine (such as Apresoline) and methyldopa (such as Aldomet) which are antihypertensive agents have been used (Patent Document 1). Further, in order to suppress eclamptic co-attack resulted by worsening of pregnancy hypertension, magnesium sulfate may be used as well.

Patent Document 1: Gazette of Japanese Patent Laid-Open No. 2000/178,240

### Disclosure of the Invention

### Problems that the Invention is to Solve

However, antihypertensive agents such as hydrazine and methyldopa are not always effective to severe gestosis. Further, in pathology of pregnancy hypertension, a fetus tries to increase its own blood pressure by promoting the secretion of angiotensin which is a vasoconstrictive peptide so as to stand up to stress. Administration of an antihypertensive agent such as hydrazine and methyldopa for a purpose of lowering the blood pressure of the mother's body has a possibility of affecting the adaptation of the fetus to stress since such a drug passes through the placenta. On the other hand, magnesium sulfate is not only toxic to the mother's body but also anxious for its side action to the fetus since it passes through the placenta.

An object of the present invention is to provide a practical therapeutic agent which solves the problems in the above-mentioned conventional therapeutic agents for hypertension, particularly, for pregnancy hypertension, is effective regardless of the content of the disease and does not affect the mother's body and the fetus when administered to a patient suffering from pregnancy hypertension.

### Means for Solving the Problems

Thus, among the present inventions, the invention mentioned in claim 1 is characterized in that it is a therapeutic agent where aminopeptidase A is an effective ingredient. The invention mentioned in claim 2 is the invention according to claim 1, wherein it is characterized in having efficacy to hypertension. The invention mentioned in claim 3 is the invention according to claim 2, wherein the hypertension is any of pregnancy hypertension, acute hypertensive encephalopathy, cerebral hemorrhage, acute cardiac insufficiency and acute aortic dissection. The invention mentioned in claim 4 is the invention according to claim 1, wherein it is an anti-tumor agent. The invention mentioned in claim 5 is characterized in that it is a therapeutic agent for pregnancy hypertension where protein which does not pass through placenta is an effective ingredient. The invention mentioned in claim 6 is the invention according to claim 5, wherein the protein is aminopeptidase A.

As to the aminopeptidase A (EC 3. 4. 11. 7; it is also called angiotensinase; hereinafter, it will be referred to as APA protein), that which is collected from human body (including a purified product) or that which is artificially produced may be advantageously used. As to a process for producing the APA protein, there may be adopted a process where 71 amino acids from the N terminal are substituted with a signal peptide (i.e., signal peptide of human trypsin II or human A-LAP) of SEQ ID No. 3 (corresponding to SEQ ID No. 1) or SEQ ID No. 4 (corresponding to SEQ ID No. 2) transcribed/translated from DNA as shown by SEQ ID No. 1 or SEQ ID No. 2 which will be mentioned later whereby a change from an inherent cell membrane bonding type to an extracellular secretion type is done. In accordance with such a producing process, it is now possible to produce a very highly pure APA protein.

### Advantages of the Invention

According to the therapeutic agent of the present invention, an APA protein which is the effective ingredient metabolizes angiotensin II which is a vasoconstrictive peptide and decomposes into angiotensin III whereby a mean blood pressure is able to be effectively lowered. Accordingly, it is able to be advantageously used as a therapeutic agent for various hypertensions such as pregnancy hypertension and also for hypertensive crisis such as acute hypertensive encephalopathy, cerebral hemorrhage, acute cardiac insufficiency and acute aortic dissection. Further, since the therapeutic agent for hypertension according to the present invention comprises an APA protein which is abundantly present in human blood and organs as an effective ingredient, there is almost no possibility of producing an antibody even when it is administered to human whereby its side effects to human body is very small. Furthermore, an APA protein which is an effective ingredient has large molecular weight and does not pass through placenta and, therefore, there is no such an anxiety in the treatment of pregnancy hypertension that it has a harmful effect to fetus due to lowering of blood pressure of fetus or has a side effect to fetus.

It has been also clarified that angiotensin II participates in generation, growth and progress of tumor via angiogenesis, etc. of tumor cells but the therapeutic agent of the present invention has a function of decomposing angiotensin II and, therefore, it also achieves an efficacy to various cancers such as uterine cervical cancer, choriocarcinoma, endometrial carcinoma, ovarian cancer, breast cancer, prostatic cancer, fibrosarcoma, kidney cancer, stomach cancer, liver cancer, colorectal cancer, lung cancer, glioma and melanoma. Incidentally, it has already been known that gene of an APA protein has an anti-tumor action (refer to Laboratory Investigation, (2004) 84, 639 to 648).

### Best Mode for Carrying Out the Invention

As hereunder, an embodiment of the therapeutic agent of the present invention will be illustrated in detail on the basis of drawings, etc.

### [Process for production of human APA protein]

In this embodiment, an APA of a secretion type prepared by substituting the amino acid sequence until the 71st one from the N terminal with a signal peptide of human trypsin II represented by SEQ ID No. 3 or of human A-LAP represented by SEQ ID No. 4 was incubated utilizing insect cells (such as cells of silkworm) whereupon human APA protein was produced. As hereunder, the process for producing it will be mentioned in detail.

(a) Amplification of cDNA (the DNA represented by SEQ ID Nos. 1 and 2) coding for an APA of a secretion type; and construction of transfer vector
Construction of cDNA coding for an APA of a secretion type was conducted by means of a PCR method where cDNA coding for human APA protein existing in nature was used as a template. PCR (Polymerase Chain Reaction) is a reaction where the reaction of DNA polymerase is carried out continuously and repeatedly whereby DNA fragments sandwiched between a set of primers are specifically amplified.

Two primers (sense and antisense primers) were used for PCR. The sense primer encodes human trypsin II-signal peptide (MNLLLILTFVAAAVAA) and human APA (Ala⁷²-Asp⁷⁶) following a precursor sequence of tropomyosin of lobster(AACTCCTAAAAAACCGCCACC). The antisense primer encodes human APA Leu⁹⁵³-Gly⁹⁵⁷ with six histidine residues at its C-terminus. The PCR reaction was carried out with both primers for 1 cycle at 98 °C for 3.5 min, followed by 30 cycles at 94 °C for 0.5 min, at 52 °C for 0.5 min, and at 72 °C for 3 min. The amplified DNA fragment was separated by means of electrophoresis using agarose gel and the aimed DNA fragment was recovered and purified from the gel by a conventional method.

After that, the DNA fragment purified by a pENTR-/D-TOPO Cloning Kit of Invitrogen was integrated with a pENTR plasmid. Further, cDNA of APA of a secretion type was integrated with pDEST8 (Invitrogen) which was a transfer vector to baculovirus using a Gateway LR clonase (Invitrogen) to construct a transfer vector.

(b) Preparation of a secretion-type APA-expressing baculovirus
Since *Escherichia coli* is a prokaryote, it often happens that a correct folding is not available when a protein derived from a eukaryote such as animals (such as human APA protein) is expressed using the expression system. Therefore, we paid our attention to a baculovirus-insect cell system as a highly expressing system using eukaryotic cells. As compared with a mammalian cell system, the baculovirus expression system is very cheap and, in addition, operation, etc. for the incubation are not troublesome whereby its usability is very good. Moreover, there is a characteristic that the aimed protein is able to be expressed in large quantities and also in a state where the intrinsic biological nature is still retained.

In the baculovirus expressing system as such, baculovirus in the field of recombinant proteins, for example, stands for a double-stranded DNA virus of insects which is called a nuclear polyhedrosis virus (NPV). The virus in this group has a characteristic that an inclusion body called a polyhedron comprising protein called a polyhedrin in nucleus of the infected cells is produced in large quantities. Now, it is a baculovirus expression system where the very strong promoter of this polyhedrin gene is utilized so that synthesis of a recombinant protein is conducted within insect cells.

That which has been commonly used as such a baculovirus expressing system at present is a system using baculovirus of noctuid-moths family, Autographa californica NPV (AcNPV) as a vector and Sf9 derived from larva of Spodoptera Frugiperda belonging to the same noctuid-moths family as infected cells. Besides the above, a system using BmNPV which is a nuclear polyhedrosis virus of silkworm (*Bombix mori*) has been also developed in Japan. In the latter system utilizing the silkworm, there is an advantage that, even when a large-volume incubation using a medium containing expensive serum is not used, the product is able to be produced in large quantities by a direct inoculation of a recombinant virus to larva of silkworm. Incidentally, in the production of the APA protein of the present invention, any of the above systems is applicable.

Further, preparation of a recombinant-type virus will be illustrated. Since gene of baculovirus is as long as 130 kb, it is not possible to directly insert the gene which is to be expressed into the downstream of a polyhedrin promoter in the case of a common DNA operation which is conducted in test tubes. However, when the Bac-to-Bac System of Invitrogen is utilized, it is now possible to prepare a recombinant virus easily and quickly. Accordingly, as mentioned above, the aimed DNA fragment is once integrated with a plasmid called a transfer vector and, after that, it is introduced into E. *coli* DH10Bac (Invitrogen) to prepare a bacmid DNA containing a cDNA of a secretion-type APA.

In the Bac-to-Bac system, gene which is to be expressed is firstly integrated with a donor plasmid having the sequences of both ends of transposon Tn7 of *E*. *coli* and is then introduced into *E*. *coli* DH10Bac having a baculovirus DNA (baculovirus shuttle vector = bacmid) with which a target site of Tn7 and a replication origin in *E*. *coli* were integrated. A helper plasmid which expresses a transposase was also introduced into the bacteria and, in the cells, a region including the aimed gene of the donor plasmid is transferred to a bacmid by the action of the transposase whereupon a bacmid of recombinant type is able to be prepared. When the bacmid is purified from *E. coli* and transfected to insect cells, virus is prepared in the insect cells. In this method, insertion of exogenous gene into viral DNA is conducted in *E*. *coli* and, therefore, there are advantages that the time is able to be shortened and that the virus is already cloned in this stage whereby operation (purification) to the aimed virus except to the virus of a non-recombinant type is not necessary.

The bacmid DNA of a human secretion-type APA prepared as such was introduced into Sf9 cells using a Cellfection Reagent (Invitrogen) and said cells were incubated at 27°C for 3 days to prepare virus. After that, the virus secreted into the supernatant liquid of the culture was recovered and amplified twice to prepare a virus solution of high titer.

(c) Expression of human APA protein
The virus of a recombinant type prepared as above was increased by means of scaling it up to prepare a virus stock of high titer. When too many subcultures are conducted in scaling up, variant is generated and expression efficiency lowers and, therefore, it is preferred not to subculture for 6 or more generations. Although the resulting virus stock is able to be semipermanently preserved at -80°C, its titer lowers due to freezing and thawing. Accordingly, since it is able to be preserved for about two years even at 4°C, it is preferred that the using one is not frozen.

For a purpose of efficient expression of protein, it is necessary that each cell is infected with viruses of as many as possible. Therefore, MOI (multiplicity of infection) is made about 5 to 10. Further, infection is not conducted when the cell density becomes very high but is conducted at the stage of a logarithmic growth phase. Although expression of the secreted APA is usually reaches its peak after 48 to 72 hours from the infection, it was anyhow observed by taking a time course.

In this embodiment, Sf9 cells were incubated under the condition of 27°C in a serum-free medium SFM 900 II (Invitrogen) using a three-liter spinner flask by which the enzyme concentration was able to be controlled to 8 ppm. When the cell concentration reached 1.5 × 10⁵ cells/mL, the high-titer virus solution prepared as above was added within such an extent that the ratio of the virus to the cells became about 10:1 (MOI = 10) whereby a virus infection was conducted. After the infection with virus, incubation was further continued for 3 days to express the protein.

(d) Purification of human APA protein
The above culture liquid (3 liters) was recovered and separated into cells and a supernatant liquid of the culture using a centrifugal separator. The resulting supernatant liquid of the culture of the cells containing human APA protein separated therefrom was dialyzed against a Tris-HCl solution (pH 8.0) under the condition of 4°C for 36 hours at 50 mM. As to a method for purification, affinity chromatography was used.

After the purification by an anion-exchange column chromatography (DEAE Toyo Pearl; Tosoh), purification was conducted by a metal chelate column chromatography (Chelating Sepharose; Amersham Bioscience) using cobalt ion whereupon a human APA protein was prepared.

### [Administration Experiment 1]

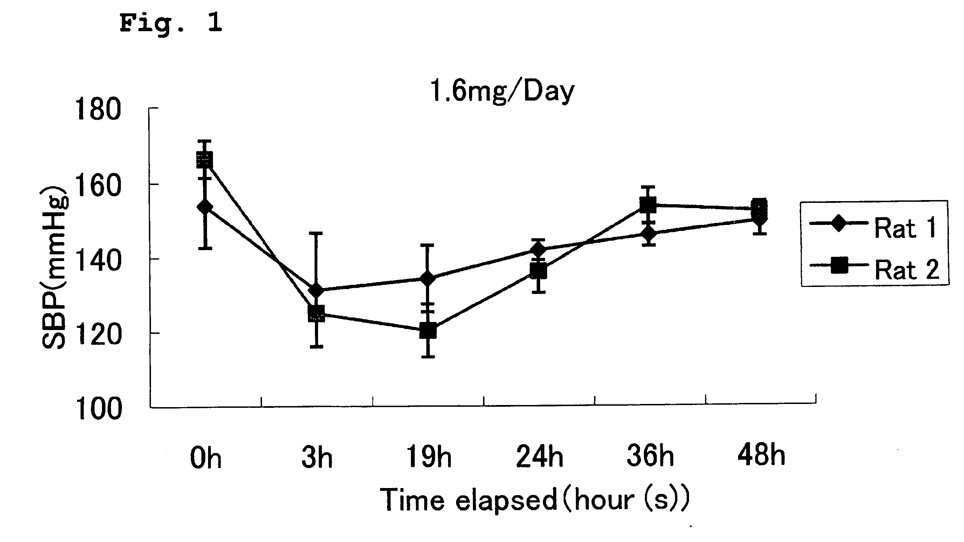

The resulting APA protein was administered to two spontaneously hypertensive rats (ten weeks old; in both rat 1 and rat 2, body weight was about 300 g each) and changes in the systolic blood pressure were checked. Initial blood pressure of the rat 1 was 155 mmHg while initial blood pressure of the rat 2 was 167 mmHg. Bolus intravenous injections of APA were conducted at doses of 1.6 mg/kg/day, and blood pressure was measured by tail-cuff method. Changes in the blood pressure after administration of the APA protein are shown in Fig. 1. Fig. 1 shows that in both of the rat 1 and the rat 2, the blood pressure was effectively lowered within short time (3 hours) by administration of the APA protein and that a low blood pressure was maintained for long time (until 48 hours).

### [Administration Experiment 2]

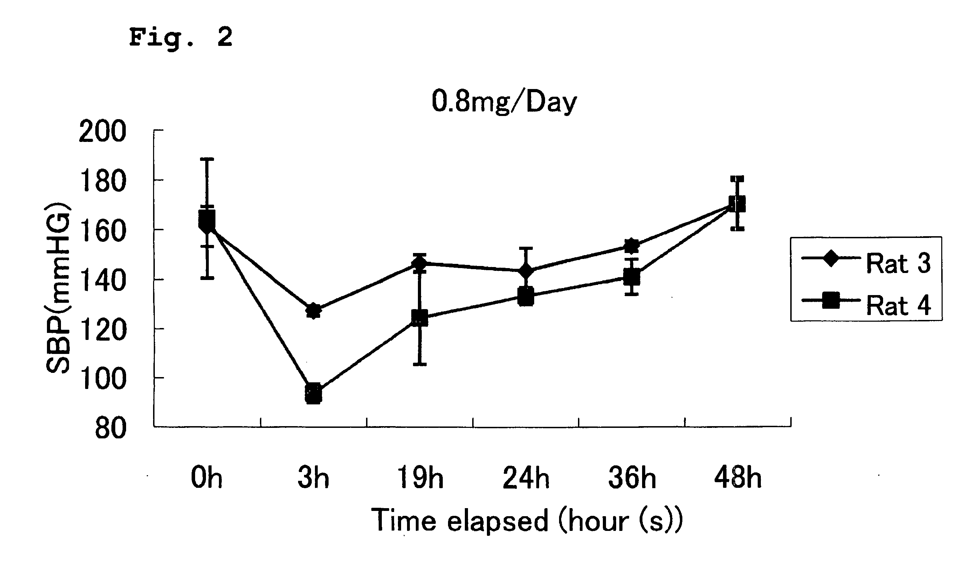

Administration experiment 2 was carried out in the same manner as in the case of the experiment 1, except that the dose of the APA protein was changed to 0.8 mg/kg/day. In the experiment 2, the initial blood pressure of rat 3 was 161 mmHg while the initial blood pressure of rat 4 was 163 mmHg. In both of the rats 3 and 4, the body weight was about 300 g each. Changes in the blood pressure after administration of the APA protein are shown in Fig. 2. Fig. 2 shows that the same as in the experiment 1, the blood pressure was effectively lowered within short time by administration of the APA protein and that a low blood pressure was maintained for long time (until 36 hours).

### [Administration Experiment 3]

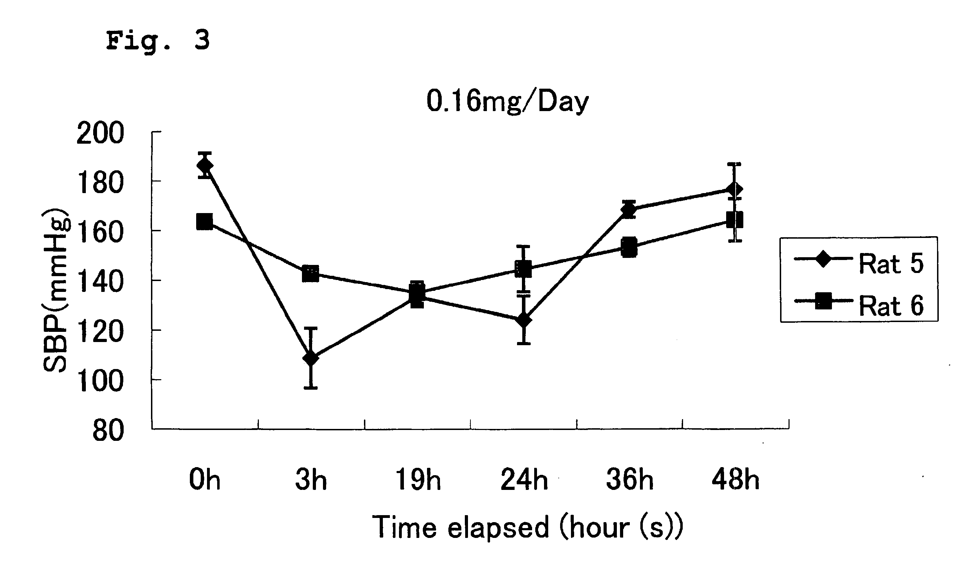

Administration experiment 3 was carried out in the same manner as in the case of the experiments 1 and 2 except that the dose of the APA protein was changed to 0.16 mg/kg/day. In the administration experiment 3, the initial blood pressure of rat 5 was 185 mmHg while the initial blood pressure of rat 6 was 164 mmHg. In both of the rats 5 and 6, the body weight was about 300 g each. Changes in the blood pressure after administration of the APA protein are shown in Fig. 3. Fig. 3 shows that, the same as in the administration experiments 1 and 2, the blood pressure was effectively lowered within short time by administration of the APA protein and that a low blood pressure was maintained for long time (until 36 hours).

### [Administration Experiment 4]

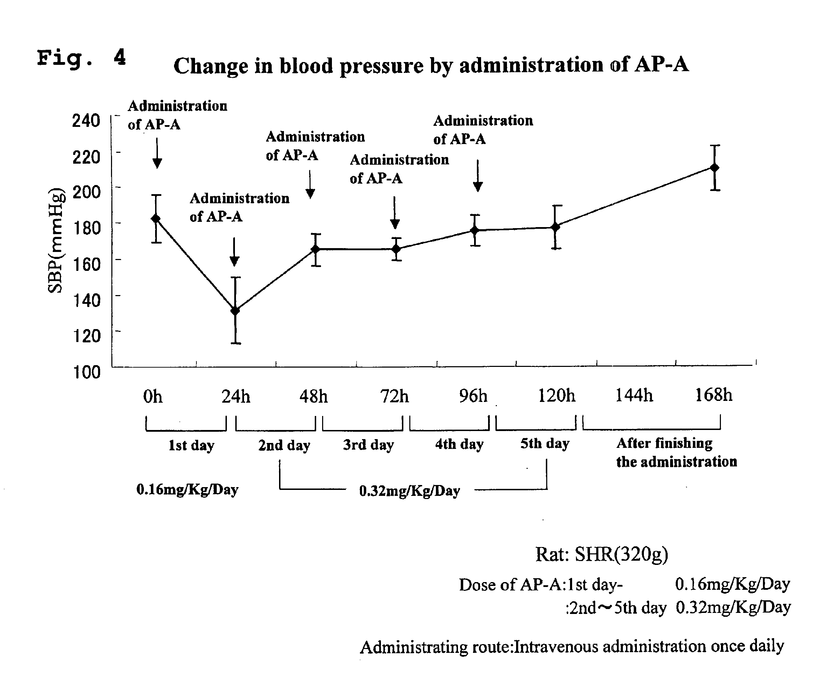

The resulting APA protein was administered to a spontaneously hypertensive rat (ten weeks old; body weight was about 320 g) intermittently and changes in the systolic blood pressure were checked. Initial blood pressure of the rat was 182 mmHg. Administration of the APA protein was conducted in such a manner that, in the first administration, 0.16 mg/kg/day was injected into the vein of the rat by mixing with PBS and that, every 24 hours from the initial administration, 0.32 mg/kg/day was repeatedly administered by the same method for four times. Measurement of blood pressure was conducted by the same manner as in the administration experiments 1 to 3. Changes in the blood pressure after administration of the APA protein are shown in Fig. 4. Fig. 4 shows that the blood pressure was effectively lowered within short time (3 hours) by administration of the APA protein and that, although the blood pressure increased thereafter, the blood pressure was maintained for long time (not shorter than 120 hours) at a lower level than that before the administration.

### [Administration Experiment 5]

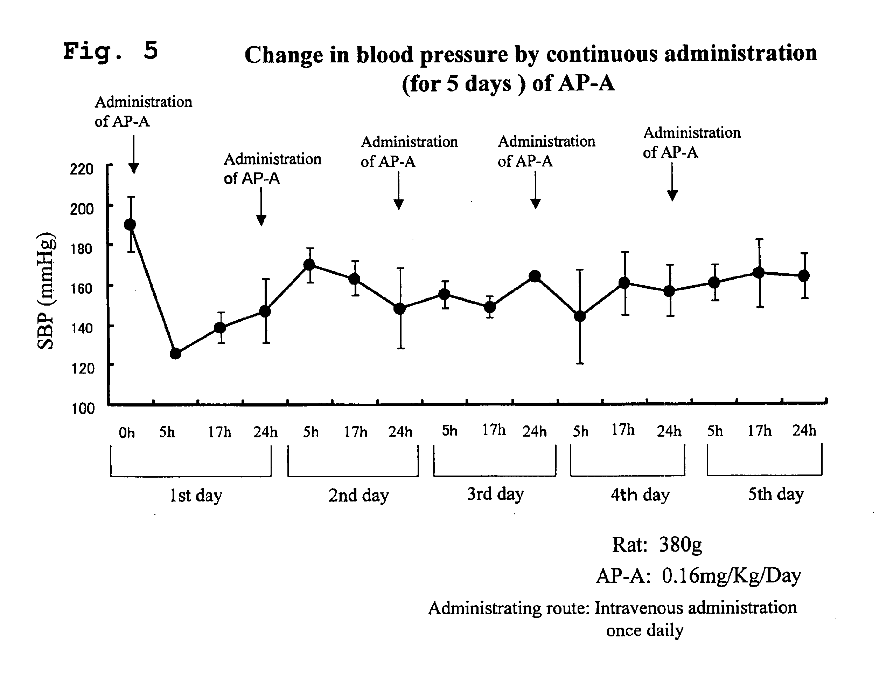

The resulting APA protein was administered to a spontaneously hypertensive rat (ten weeks old; body weight was about 380 g) intermittently and changes in the systolic blood pressure were checked. Initial blood pressure of the rat was 191 mmHg. Administration of the APA protein was conducted in such a manner that 0.16 mg/kg/day was injected into the vein of the rat by mixing with PBS and that, every 24 hours from the initial administration, the same administration was repeatedly conducted for four times. Measurement of the blood pressure was conducted by the same manner as in the administration experiments 1 to 4. Changes in the blood pressure after administration of the APA protein are shown in Fig. 5. Fig. 5 shows that the blood pressure was effectively lowered within short time (3 hours) by administration of the APA protein and that, although the blood pressure increased thereafter, the blood pressure was maintained for long time (until 144 hours) at a very lower level than that the blood pressure before the administration.

### [Administration Experiment 6]

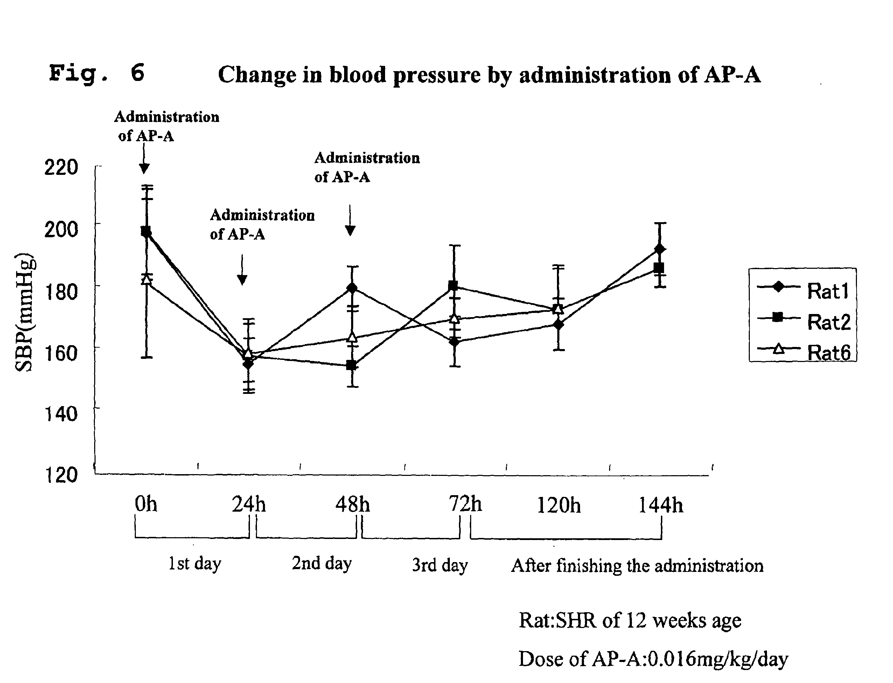

The resulting APA protein was administered to three spontaneously hypertensive rats (12 weeks old; in all of rats 7 to 9, body weight was about 300 g each) intermittently and changes in the systolic blood pressure were checked. The initial blood pressure of the rat 7 was 198 mmHg, the initial blood pressure of the rat 8 was 199 mmHg and the initial blood pressure of the rat 9 was 182 mmHg. Administration of the APA protein was conducted in such a manner that 0.016 mg/kg/day was mixed with PBS and the mixture was injected into the veins of the rats and the same administration was repeated for 24 hours for three times. Measurement of the blood pressure was conducted by the same manner as in the administration experiments 1 to 5. Changes in the blood pressure after administration of the APA protein are shown in Fig. 6. Fig. 6 shows that, by the first administration of the APA protein, the blood pressure was effectively lowered and that, although the blood pressure gradually increased thereafter, the blood pressure was maintained in a lower level for long time (not shorter than 120 hours) than the level before the administration.

### Industrial Applicability

Since the therapeutic agent of the present invention exhibits an excellent hypotensive action as mentioned above, it is able to be advantageously used as a therapeutic agent for various hypertensive diseases including pregnancy hypertension.

### Brief Description of the Drawings

[Fig. 1] Fig. 1 is a drawing where changes in the blood pressure with the lapse of time after the APA protein is administered once are plotted.
[Fig. 2] Fig. 2 is a drawing where changes in the blood pressure with the lapse of time after the APA protein is administered once are plotted.
[Fig. 3] Fig. 3 is a drawing where changes in the blood pressure with the lapse of time after the APA protein is administered once are plotted.
[Fig. 4] Fig. 4 is a drawing where changes in the blood pressure with the lapse of time after the APA protein is continuously administered are plotted.
[Fig. 5] Fig. 5 is a drawing where changes in the blood pressure with the lapse of time after the APA protein is continuously administered are plotted.
[Fig. 6] Fig. 6 is a drawing where changes in the blood pressure with the lapse of time after the APA protein is continuously administered are plotted.

## Claims

1. A therapeutic agent where aminopeptidase A is an effective ingredient.

2. The therapeutic agent according to claim 1, wherein the agent is **characterized in** having efficacy to hypertension.

3. The therapeutic agent according to claim 2, wherein the hypertension is any of pregnancy hypertension, acute hypertensive encephalopathy, cerebral hemorrhage, acute cardiac insufficiency and acute aortic dissection.

4. The therapeutic agent according to claim 1, wherein the agent is **characterized in that** it is an anti-tumor agent.

5. A therapeutic agent for pregnancy hypertension where protein which does not pass through placenta is an effective ingredient.

6. The therapeutic agent for pregnancy hypertension according to claim 5, wherein the protein is aminopeptidase A.
